(19) Europäisches Patentamt
European Patent Office
Office européen des brevets

(11) Veröffentlichungsnummer: **0 241 498 B1**

# (12) EUROPÄISCHE PATENTSCHRIFT

(45) Veröffentlichungstag der Patentschrift: **12.07.89**

(51) Int. Cl.⁴: **A 61 K 35/78**

(21) Anmeldenummer: **86905810.7**

(22) Anmeldetag: **11.10.86**

(86) Internationale Anmeldenummer: **PCT/EP 86/00584**

(87) Internationale Veröffentlichungsnummer: **WO 87/02248 (23.04.87 Gazette 87/09)**

## (54) SCHMERZSTILLENDES UND ENTZÜNDUNGSHEMMENDES ARZNEIMITTEL AUF PFLANZLICHER BASIS.

(30) Priorität: **18.10.85 DE 3537160**

(43) Veröffentlichungstag der Anmeldung: **21.10.87 Patentblatt 87/43**

(45) Bekanntmachung des Hinweises auf die Patenterteilung: **12.07.89 Patentblatt 89/28**

(84) Benannte Vertragsstaaten: **AT BE CH DE FR GB IT LI LU NL SE**

(56) Entgegenhaltungen: **Unlisted Drugs, vol. 15, no. 11, November 1963, (Chatham, New Jersey, US), "Phytodolor drop", p. 87-q**

(73) Patentinhaber: **STEIGERWALD ARZNEIMITTELWERK GMBH, Havel Strasse 5, D-6100 Darmstadt (DE)**

(72) Erfinder: **KRAUSKOPF, Jobst, Lessingstrasse 19, D-6140 Bensheim 3 (DE)**

(74) Vertreter: **Rupprecht, Klaus, Dipl.- Ing., Kastanienstrasse 18, D-6242 Kronberg (DE)**

EP 0 241 498 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1989

**Beschreibung**

Die Erfindung bezieht sich auf ein Arzneimittel mit schmerzstillender und entzündungshemmender Wirkung auf pflanzlicher Basis, insbesondere auf ein Antirheumatikum.

Antirheumatika auf pflanzlicher Basis sind grundsätzlich bekannt. Sie umfassen in der Regel eine größere Anzahl von pflanzlichen Bestandteilen als Träger ihrer Wirksamkeit. Ein solches Antirheumatikum (Zusammenstellung II) besteht aus den folgenden wäßrig/alkoholischen Auszügen mit den genannten Bestandteilen.

| | | |
|---|---|---|
| Populus tremula | (Rinde : Blätter = 1 : 2) | 40,0 ml |
| Fraxinus excelsior | (Rinde : Blätter = 1 : 2) | 5,0 ml |
| Solidago virgaurea | (Rinde : Blätter = 1 : 2) | 5,0 ml |
| Arnica montana | D3 | 10,0 ml |
| Colchicum autumnale | D3 | 10,0 ml |
| Gelsemium sempervirens | D3 | 10,0 ml |

Ein ähnliches Arzneimittel mit einem zusätzlichen Gehalt an 20 ml Aristolochia D3 ist aus Unlisted Drugs (Nov. 1963), Vol. 15, No. 11 bekannt.

Seit geraumer Zeit geht das Bestreben der Ärzte bei der Arzneimittelverordnung dahin, die Belastung von Patienten durch Arzneimittel soweit wie möglich zu beschränken. Dies gilt sowohl für die Anzahl der Arzneimittelbestandteile als auch für deren Menge. Die Belastung der Patienten in dieser Hinsicht soll gering gehalten werden, um u. U. schädliche Nebenwirkungen auf ein Mindestmaß zu begrenzen.

Die Aufgabe der Erfindung besteht somit darin, ein Arzneimittel der eingangs genannten Art zu schaffen, das zum einen in seinem therapeutischen Effekt mit chemischen Arzneimitteln vergleichbar ist, zum anderen aber weitestgehend keine Nebenwirkungen hervorruft.

Überraschenderweise wurde gefunden, daß ein Arzneimittel auf pflanzlicher Basis mit Auszügen oder Inhaltsstoffen aus Populus tremula, Solidago virgaurea und Fraxinus excelsior, die der Erfindung zugrundeliegende Aufgabe löst.

Das erfindungsgemäße Arzneimittel zeichnet sich durch eine Verringerung der Anzahl der als Träger der Wirksamkeit in Frage kommenden Inhaltsstoffe gegenüber bekannten Produkten auf pflanzlicher Basis aus. Seine therapeutische Wirkung tritt besonders dann ein, wenn die erfindungsgemäß vorgegebene Abstimmung seiner Inhaltsstoffe eingehalten wird. Es zeigt eine sehr gute Verträglichkeit. Es handelt sich mithin um ein überlegenes Produkt, dessen vorteilhafte Wirkung insbesondere ohne Steigerung der einzunehmenden Dosis gegenüber bekannten Arzneimitteln mit einer größeren Anzahl von Bestandteilen erzielt wird.

Mit dem erfindungsgemäßen Phytopharmakon lassen sich bei wesentlich niedrigerem Risiko therapeutische Effekte erreichen, die in der Größenordnung derjeniger hochdosierter chemischer (nichtsteroidaler, z. B. Indomethacin) Antirheumatika liegen (vergl. Fig. 2).

Die antiphlogistische sowie analgetische, d. h. antirheumatische Wirkung des erfindungsgemäßen Arzneimittels wird mit den folgenden Versuchsergebnissen dokumentiert.

Die Versuche wurden mit der folgenden erfindungsgemäßen Zusammenstellung (Zusammenstellung I) durchgeführt: wäßrig äthanolische Auszüge aus:

| | |
|---|---|
| Popolus tremula | 47 Vol.-% Äthanol |
| Fraxinus excelsior | 50 Vol.-% Äthanol |
| Solidago virgaurea | 43 Vol.-% Äthanol. |

Die Einzelextrakte wurden aus Frischeflanzen hergestellt. Das bedingt, daß die Alkoholkonzentration um ± 2,0 % schwanken kann.

In einigen Fällen wurde an Hand von Tierversuchen die bekannte Zusammensetzung (Zusammenstellung II) in die Untersuchungen mit einbezogen.

**I Tierversuch**

Zur antiphlogistischen Wirkung der Zusammenstellung I (Dextran-Rattenpfotenödem)

1. Einleitung

Die Zusammenstellungen I und II enthalten Pflanzenwirkstoffe, die sich durch eine antipyretische, analgetische und antirheumatische Wirkung auszeichnen und sich deshalb besonders zur Behandlung akuter und subakuter rheumatischer Erkrankungen eignen. In den vorliegenden Untersuchungen wurde die antiphlogistische bzw. antiexudative Eigenschaft der Präparate im Rattenpfotenödem (Dextranödem)-Modell geprüft. Indomethacin diente als Vergleichssubstanz und positive Kontrolle.

2. Methoden

2.1 Tiermaterial

Männliche Sprague Dawley Ratten, SPF, Sut (Süddeutsche Versuchstierfarm), Körpergewicht um 200 g, Haltung 5 Tiere/Makrolonkäfig Typ IV im vollklimatisierten Raum, 23 ± 1°C, 55 ± 5 % rel. Luftfeuchtigkeit, $12/12^h$ Hell-Dunkel-Rhythmus, Leitungs-Wasser und Futter (Altromin Diät 1324) ad libitum.

2.2 Substanzen
Zusammenstellung II, Chargen-Nr. 3511, entalkoholisiert und mit Wasser aufgefüllt;
Zusammenstellung I, Chargen-Nr. 4014, entalkoholisiert und mit Wasser äufgefüllt;
Dextran-Lösung. Macrodex® 6 %, Fa. Knoll AG
Indomethacin Bestell-Nr. 7378, Fa. Sigma
$NaHCO_3$ Bestell-Nr. 6323, Fa. E. Merck.

2.3 Gruppierung
Randomisierte Zuteilung der Tiere in den Gruppen, jeweils 10 Tiere pro Gruppe:

Gruppe 1: Kontrolle 0,1 M $NaHCO_3$ 10,0 ml/kg KG
Gruppe 2: Zusammenstellung II 1,0 ml/kg KG; Verdünnung 1 : 10 mit 0,111 M $NaHCO_3$-Lösung
Gruppe 3: Zusammenstellung II 5,0 ml/kg KG; Verdünnung 1 : 2 mit 0,2 M $NaHCO_3$-Lösung
Gruppe 4: Zusammenstellung I 1,0 ml/kg KG; Verdünnung wie in Gruppe 2
Gruppe 5: Zusammenstellung I 5,0 ml/kg KG; Verdünnung wie in Gruppe 3
Gruppe 6: Indomethacin 4,0 mg/kg KG gelöst in 10,0 ml 0,1 M $NaHCO_3$-Lösung.

Die Verdünnung von den Prüfpräparaten wurde so vorgenommen, daß das intragastral per Schlundsonde applizierte Volumen 10 ml/kg KG betrug.

2.4 Durchführung
16 Stunden vor dem Versuchsbeginn wurde den Tieren Futter entzogen. Die Prüfsubstanzen wurden 30 Minuten vor der Ödemsetzung verabreicht. Zur Erzeugung der Ödeme wurde 0,1 ml einer 6 %-igen Dextran-Lösung intraplantar in die rechte Hinterpfote injiziert. Unmittelbar danach wurde den Tieren das Wasser entzogen. Die Messung der Pfotenvolumina erfolgte mit dem Volumen-Meßgerät von Kemper et al[1] in µA (Empfindlichkeitsstufe II) eine Stunde vor der Ödemsetzung (Leerwert) und 1, 2, 4, 6 und 24 Stunden nach der Dextraninjektion.
Die Differenz zum Leerwert bei jeder Messung ergibt die Größe des Ödems. Die Wirkung der Prüfpräparate wird wie folgt berechnet:

$$\text{\%-Wirkung (Ödem-Hemmung)} = 100 - \frac{\bar{x}\text{ d. beh. Tiere}}{\bar{x}\text{ d. unbeh. Tiere}} \times 100$$

Der statistische Vergleich zur Kontrollgruppe erfolgte mit dem Student t-Test für unverbundene Stichproben mit zweiseitiger Fragestellung.

2.5 Ergebnisse
In Tab. 1 sind alle Ergebnisse zusammengestellt, während der Verlauf der Pfotenvolumina, relative Größe der Pfoten und Ödemhemmung in % in Fig. 1 und 2 dargestellt sind. Fig. 3 zeigt die maximal erreichte Wirkung der Prüfpräparate 6 Stunden nach der Ödeminduktion.
Bei den Versuchsgruppen Zusammenstellung II 1,0 ml, Zusammenstellung I 1,0 und 5,0 ml/kg KG sowie bei Indomethacin 4,0 mg/kg KG stieg die Hemmung des Dextranödems kontinuierlich bis zur 6. Stunde, wenn auch der Unterschied zur Kontrollgruppe nicht immer statistisch signifikant ist, während Zusammenstellung II 5,0 ml/kg KG nur in der 6. Stunde nach der Ödemsetzung eine statistisch nichtsignifikante Verminderung (23 %) des Ödems zeigte (Tab. 1).
Die Fig. 3 zeigt eine Dosisabhängigkeit der Wirkung der Zusammenstellungen sowie der Unterschied in Ödem-Hemmung zwischen der Zusammenstellung I und der Zusammenstellung II. Während die Zusammenstellung II 1,0 und 5,0 ml/kg KG eine statistisch nicht signifikante Inhibition von 19 bzw. 23 % bewirkten, wurde mit der Zusammenstellung I 1,0 und 5,0 ml/kg KG eine Hemmung von 28 ($P \leq 0,02$) und 35 % ($P \leq 0,01$) registriert. Das Vergleichspräparat Indomethacin inhibierte das Dextran-Ödem zu 43 % ($P \leq 0,001$).

3. Schlußfolgerung
In dieser Prüfung ist die Zusammenstellung I der Zusammenstellung II um 1 1/2faches als Antiphlogistikum überlegen.
Die antiphlogistische Potenz der Zusammenstellung I 1,0 ml (20 Tropfen) und besonders von 5,0 ml (100 Tropfen) mit 35 %-iger Ödemhemmung ($ED_{35}$) reicht nahe an die Wirkung von 4 mg Indomethacin/kg KG ($ED_{43}$) heran; demzufolge ist die Zusammenstellung I als ein gut wirksames antiphlogistisches Pytotherapeutikum anzusehen.

**Literatur**

[1] Kemper, F. und Ameln, G.
eine neue elektrische Methode zur Messung von Volumina, Druck und anderem sowie ein Beispiel ihrer Anwendung
Z. ges. exp. Med. Bd. 131, 407-411 (1959).

**Tabelle 1**

Zusammenfassung der Ergebnisse
(a) Volumenzunahme der Pfotenödeme in µA x ± s  n = 10
(b) Größe der Entzündung in %
(c) Entzündungshemmung in %

| | | | O-Wert | 1 h | 2 h | 4 h | 6 h | 24 h |
|---|---|---|---|---|---|---|---|---|
| 1. | Kontrolle | (a) | 19,2 | 44,5 | 35,3 | 23,1 | 15,9 | 1,7 |
| | | | ± 1,6 | ± 9,7 | ± 7,9 | ± 4,5 | 7 | ± 1,6 |
| | | (b) | | 100 | 100 | 100 | 100 | 100 |
| | | (c) | | - | - | - | - | - |
| 2. | Z II | (a) | 19,6 | 39,5 | 32,0 | 19,0 | 12,9 | ± 0,0 |
| | entalkohol. | | ± 1,3 | ± 10,2 | ± 9,0 | ± 5,7 | ± 3,1 | ± 2,0 |
| | 1 ml/kg KG | | | | | | | |
| | | (b) | | 88,8 | 90,7 | 82,3 | 81,2 | 0,0 |
| | | (c) | | 11,2 | 9,3 | 17,7 | 18,8 | 100,0 |
| 3. | Z II | (a) | 19,6 | 45,0 | 33,6 | 22,7 | 12,2 | 0,6 |
| | entalkohol. | | ± 1,1 | ± 11,4 | ± 10,7 | ± 5,3 | | ± 1,8 |
| | 5 ml/kg KG | | | | | | | |
| | | (b) | | 101,1 | 95,2 | 98,3 | 76,8 | 35,3 |
| | | (c) | | ± 1,1 | 4,8 | 1,7 | 23,2 | 64,7 |
| 4. | Z I | (a) | 19,6 | 42,8 | 30,6 | 18,7* | 11,4* | ± 0,0 |
| | entalkohol. | | ± 1,4 | ± 6,9 | ± 4,5 | ± 2,8 | ± 2,0 | ± 1,4 |
| | 1 ml/kg KG | | | | | | | |
| | | (b) | | 96,2 | 86,7 | 81,0 | 71,7 | 0,0 |
| | | (c) | | 3,8 | 13,3 | 19,0 | 28,3 | 100,0 |
| 5. | Z I | (a) | 21,0 | 43,7 | 31,8 | 19,8 | 10,4** | - 0,5 |
| | entalkohol. | | ± 1,1 | ± 9,3 | ± 7,4 | ± 5,2 | ± 2,8 | ± 2,0 |
| | 5 ml/kg KG | | | | | | | |
| | | (c) | | 1,7 | 9,9 | 14,2 | 34,5 | -129,4 |
| 6. | Indomethacin | (a) | 20,6 | 41,7 | 27,9 | 15,5*** | 9,1*** | 0,4 |
| | Fa. Sigma | | ± 1,6 | ± 7,8 | ± 5,4 | ± 2,5 | ± 2,6 | ± 1,3 |
| | Bestell-Nr. 7378 | | | | | | | |
| | | (b) | | 93,8 | 79,1 | 67,1 | 57,3 | 23,6 |
| | | (c) | | 6,2 | 20,9 | 32,9 | 42,7 | 76,4 |

* P ⩽ 0,02  Z I = Zusammenstellung I
** P ⩽ 0,01  Z II = Zusammenstellung II
*** P ⩽ 0,001

**II Humanversuche**

Untersuchung 1:

Zur Auswertung kamen 20 Patienten, 10 Männer und 10 Frauen. Das durchschnittliche Alter betrug 46,3 ± 2,1 Jahre, das durchschnittliche Gewicht 72,8 ± 2,7 kg. Der Broca-Index zeigt mit 108,6 ein leichtes Übergewicht an. Die Krankheitsdauer betrug zwischen einem und 33 Jahren. Keine Vortherapie erfolgte in 13 Fällen, während in 7 Fällen mit 3 verschiedenen, häufig verwendeten, nichtsteroidalen Antirheumatika vortherapiert wurde. 4 x mit Voltaren®, 1 x mit Musaril®, 1 x mit Arumalon® und 1 x mit Injektionen. Die eingeschlossenen Diagnosen waren 6 x Arthrosis deformans, 9 x HWS-Syndrom, 18 x Periarthritis humeroscapularis, 12 x Epikondylitis humeri. Hinzu kommen 10 sonstige Angaben (Mehrfachnennung möglich).

Die Dosis betrug in allen Fällen 3 x 30 Tropfen pro Tag.

Die Abschlußbeurteilung lautet: Sehr gut 7 x, gut 8 x, ausreichend 3 x, nicht ausreichend 1 x, nicht beurteilbar 1 x.

In den Abstufungen 1 bis 4 (1 = nicht vorhanden, 2 = gering, 3 = mäßig, 4 = stark) wurden folgende Erhebungen dokumentiert:

A) Feststellungen des Arztes

A1) Bewegungsschmerz

| | |
|---|---|
| vor Therapie | 3,4 ± 0,6 |
| nach 1 woche | 2,8 ± 0,6 |
| nach 2 Wochen | 2,3 ± 0,6 |
| nach 4 Wochen | 2,1 ± 0,6 |

A2) Dauerschmerz

| | |
|---|---|
| vor Therapie | 2,9 ± 0,6 |
| nach 1 Woche | 2,4 ± 0,8 |
| nach 2 Wochen | 2,1 ± 0,6 |
| nach 4 Wochen | 1,8 ± 0,8 |

A3) Bewegungseinschränkung

| | |
|---|---|
| vor Therapie | 3,6 ± 0,7 |
| nach 1 Woche | 2,8 ± 0,6 |
| nach 2 Wochen | 2,5 ± 0,6 |
| nach 4 Wochen | 2,3 ± 0,7 |

B) Feststellungen des Patienten (angegeben als Abstand einer Strichmarkierung vom Ursprung einer 8 cm langen Linie - Größerwerden der Linie bedeutet Abnahme der Beschwerden).

B1) Bewegungsschmerz

| | |
|---|---|
| vor Behandlung | 2,0 ± 1,9 |
| nach 1 Woche | 4,1 ± 2,1 |
| nach 2 Wochen | 5,2 ± 1,8 |
| nach 4 Wochen | 5,8 ± 1,7 |

B2) Dauerschmerz

| | |
|---|---|
| vor Behandlung | 3,9 ± 2,0 |
| nach 1 Woche | 5,1 ± 2,1 |
| nach 2 Wochen | 5,9 ± 1,9 |
| nach 4 Wochen | 6,5 ± 1,9 |

B3) Bewegungseinschränkung

| | |
|---|---|
| vor Behandlung | 1,5 ± 2,3 |
| nach 1 Woche | 3,7 ± 2,3 |
| nach 2 Wochen | 4,9 ± 2,2 |
| nach 4 Wochen | 5,7 ± 1,8 |

**Laborwerte** (Mittelwerte)

| | Ausgangswert | 2 Wochen | 4 Wochen |
|---|---|---|---|
| SGOT | 11,0 | 9,0 | 8,6 |
| SGPT | 15,8 | 14,1 | 12,7 |
| - GT | 35,8 | 29,7 | 30,2 |
| AP | 105,6 | 107,6 | 108,0 |
| Harnsäure | 4,0 | 3,9 | 3,6 |
| Harnstoff | 32,0 | 29,8 | 31,2 |
| Kreatinin | 0,92 | 0,87 | 0,87 |
| Glukose | 77,2 | 79,2 | 79,7 |
| Leukozyten | 5,1 | -- | 6,1 |
| Erythrozyten | 4,7 | -- | 4,5 |
| HB | 14,5 | -- | 14,8 |
| Thrombozyten | 144,7 | -- | 149,9 |
| BSG | | | |
| 1. Stunde | 7,1 | -- | 8,2 |

| | | | |
|---|---|---|---|
| 2. Stunde | 20,1 | -- | 21,4 |

Die Urinbefunde zeigten keine Verschlechterungen. Nebenwirkungen wurden nicht beobachtet.

**Beurteilung**

Die Beurteilung der ausschließlich mit Zusammensetzung I pharmakotherapierten Patienten erbringt in fünfzehn von zwanzig Fällen gute oder sehr gute Ergebnisse. Die Feststellungen des Arztes ebenso wie die der Patienten begründen dies mit dem Nachweis der Verminderung der Beschwerden. In ähnlichem Ausmaß wären Effekte bei der Verwendung chemischer nicht steroidaler Antirheumatika zu erwarten gewesen. Allerdings liegt die Rate unerwünschter Wirkungen bei diesen Präparaten wesentlich höher. Durch Zusammensetzung I wurden in keinem Fall Nebenwirkungen beobachtet. Für eine vierwöchige ununterbrochene Therapie ist dies ein sehr gutes Ergebnis.

**Untersuchung 2:**

Ausgewertet wurden 31 Fälle, 10 Männer und 21 Frauen. Das durchschnittliche Alter betrug 59,8 Jahre, das durchschnittliche Gewicht 68,9 kg. Der Broca-Index betrug 101,9.
Die Diagnosen setzen sich wie folgt zusammen:

9 x chronische Polyarthritis,
11 x Arthrosis deformans,
4 x LWS-Syndrom,
5 x HWS-Syndrom,
2 x "Sonstiges"
(beide Male Borbus Bechterew).

Als Vortherapie wurden sieben verschiedene, häufig verwendete nichtsteroidale Antirheumatika (NSAR) verwendet. Sieben Patienten erhielten keine Vortherapie.
Die Dosis 3 x 30 Tropfen erhielten 14 Patienten, 3 x 40 Tropfen 17 Patienten.
2 Patienten brachen die Behandlung ab: 1 Patient wegen eines entgleisten Diabetes, 1 Patient wegen exacerbierter Synovitis.
Unter der Rubrik Nebenwirkung wird der entgleiste Diabetes genannt, der Zusammenhang ist nicht eindeutig. Andere Nebenwirkungen werden nicht genannt.
24 der 31 Patienten erhielten keine Zusatzmedikation.
In den Abstufungen 1 - 4 (1 = nicht vorhanden, 2 = gering, 3 = mäßig, 4 = stark) wurden folgende Erhebungen dokumentiert:

A) Feststellungen des Arztes
Beschwerdescore

| | |
|---|---|
| vor Therapie | 3,2 ± 0,5 |
| nach 1 Woche | 2,7 ± 0,6 |
| nach 2 Wochen | 2,4 ± 0,8 |
| nach 4 Wochen | 2,0 ± 0,7 |

Der Score umfaßt die Feststellungen des Arztes, zusammengesetzt aus Bewegungsschmerz, Dauerschmerz und Bewegungseinschränkung.

Beschwerden im einzelnen:

A1) Bewegungsschmerz

| | |
|---|---|
| vor Therapie | 3,1 ± 0,7 |
| nach 1 Woche | 2,6 ± 0,8 |
| nach 2 Wochen | 2,2 ± 0,9 |
| nach 4 Wochen | 1,7 ± 0,9 |

A2) Dauerschmerz

| | |
|---|---|
| vor Therapie | 3,2 ± 0,5 |
| nach 1 Woche | 2,8 ± 0,6 |
| nach 2 Wochen | 2,3 ± 0,8 |
| nach 4 Wochen | 2,1 ± 0,8 |

A3) Bewegungseinschränkung

| | |
|---|---|
| vor Therapie | 3,4 ± 0,6 |
| nach 1 Woche | 2,7 ± 0,8 |
| nach 2 Wochen | 2,6 ± 1,0 |
| nach 4 Wochen | 2,3 ± 1,1 |

B) Der Score der Beurteilung des Zustandes durch den Patienten selbst, berechnet aus den Einzelsymptomen Bewegungsschmerz, Dauerschmerz und Bewegungseinschränkung ergibt folgende Werte: (Minimum 0 = starke Beschwerden, Maximum 8 = keine Beschwerden).

Beschwerdescore nach Feststellung des Patienten

| | |
|---|---|
| vor Therapie | 2,6 ± 1,1 |
| nach 1 Woche | 3,6 ± 1,1 |
| nach 2 Wochen | 4,5 ± 1,4 |
| nach 4 Wochen | 5,2 ± 1,3 |

B1) Bewegungsschmerz

| | |
|---|---|
| vor Therapie | 2,8 ± 1,5 |
| nach 1 Woche | 3,8 ± 1,4 |
| nach 2 Wochen | 4,8 ± 1,7 |
| nach 4 Wochen | 5,7 ± 1,6 |

B2) Dauerschmerz

| | |
|---|---|
| vor Therapie | 2,4 ± 1,1 |
| nach 1 Woche | 3,5 ± 1,1 |
| nach 2 Wochen | 4,6 ± 1,2 |
| nach 4 Wochen | 5,2 ± 1,3 |

B3) Bewegungseinschränkung

| | |
|---|---|
| vor Therapie | 2,5 ± 1,2 |
| nach 1 Woche | 3,5 ± 1,3 |
| nach 2 Wochen | 4,2 ± 1,7 |
| nach 4 Wochen | 4,8 ± 1,9 |

**Laborwerte** (Mittelwerte)

| | Ausgangswert | 2 Wochen | 4 Wochen |
|---|---|---|---|
| SGOT | 15,7 | 15,4 | 15,9 |
| SGPT | 16,2 | 16,7 | 15,8 |
| - GT | 14,5 | 15,2 | 15,7 |
| AP | 153,9 | 157,4 | 155,4 |
| Harnsäure | 4,2 | 4,3 | 4,2 |
| Harnstoff | 16,8 | 17,6 | 17,3 |
| Kreatinin | 1,0 | 1,0 | 1,0 |
| Blutzucker | 92,8 | 88,5 | 88,3 |
| Kalium | 4,2 | 4,2 | 4,3 |
| Hämatokrit | 41,0 | - | 41,6 |
| Leukozyten | 5,8 | - | 5,7 |
| Erythrozyten | 4,4 | - | 4,5 |
| HB | 13,5 | - | 14,1 |
| Thrombozyten | 244,8 | - | 243,4 |
| BSG | | | |
| 1. Stunde | 16,4 | - | 12,4 |
| 2. Stunde | 27,9 | - | 20,0 |

Die Urinbefunde zeigten keine Hinweise auf Verschlechterungen.

Ergebnisse aufgrund der teststatischen Bearbeitung

Entsprechend dem Typ der Untersuchung 2 als Beobachtungsstudie wurden im explorativen Sinn t-Tests für verbundene Stichproben nach trimming der Extrema der Verteilung gerechnet. Eine Differenz wurde dann als statistisch von Null verschieden betrachtet, wenn deren p-Wert unter einem kritischen von 0,05 lag.

Danach ergibt sich für die Scores der klinischen Symptomatik:

alle Differenzen sind mit einem p = 0.0000 von Null verschieden, so daß von einer deutlichen Besserung sowohl im Urteil des Arztes als auch in dem des Patienten ausgegangen werden muß.

Für die Reihe der Laborwerte ist folgendes festzustellen:

- Anstieg des Harnstoffes von 0 nach 2 um 0,8 mg/dl $p = 0{,}0192$
- Anstieg des Hb um 0,4 g/dl von 0 nach 4, $p = 0{,}0004$
- Abfall der BSG 1. Stunde um 3,3 E von 0 nach 4, $p = 0{,}0193$
- Abfall der BSG 2. Stunde um 6,7 E von 0 nach 4 $p = 0{,}117$

Die oben mitgeteilten Veränderungswerte sind Mittelwertangaben.

**Beurteilung**

Die berichteten Ergebnisse zeigen, daß eine Reduktion der subjektiven Beschwerden (Schmerz) und auch der Bewegungseinschränkung in einem relevanten Ausmaß eingetreten ist. Eine vollständige Heilung ist bei der vorliegenden Erkrankung unmöglich. Die umfangreiche Literatur über die Behandlung der hier untersuchten Krankheitsbilder mit chemischen, nicht steroidalen Antirheumatika enthält Ergebnisse, die den hier mit der Zusammenstellung 1 erreichten entsprechen.

Von besonderer Wichtigkeit ist die Verringerung der Blutkörperchensenkungsgeschwindigkeit (signifikant), die ein Maß für die systemische entzündliche Komponente der vorliegenden Krankheit ist. Seitens der pharmazeutischen Industrie werden große Bemühungen unternommen, um diesen Effekt auch mit anderen Produkten zu erreichen.

Sowohl die Laborwerte als auch die ärztlichen Berichte zeigen, daß unerwünschte Wirkungen allenfalls in fraglichem Zusammenhang mit der Anwendung der Zusammensetzung I (entgleister Diabetes) aufgetreten sind. Aus ärztlicher Sicht ist der Zusammenhang mindestens fraglich.

**Patentansprüche**

1. Arzneimittel mit schmerzstillender und entzündungshemmender Wirkung auf pflanzlicher Basis mit Auszügen oder Inhaltsstoffen aus
Populus tremula
Solidago virgaurea
Fraxinus excelsior
als alleinige Wirkstoffe.

2. Arzneimittel nach Anspruch 1, dadurch gekennzeichnet, daß es Auszüge oder Inhaltsstoffe bestehend aus
50 - 70 Vol.-% Populus tremula
10 - 30 Vol.-% Solidago virgaurea
10 - 30 Vol.-% Fraxinus excelsior
aufweist.

3. Arzneimittel nach Anspruch 1 oder 2, dadurch gekennzeichnet, daß es Auszüge oder Inhaltsstoffe bestehend aus
60 Vol.-% Populus tremula
20 Vol.-% Solidago virgaurea
20 Vol.-% Fraxinus excelsior
aufweist.

4. Arzneimittel nach einem der Ansprüche 1 bis 3, dadurch gekennzeichnet, daß in dem Auszug oder Inhaltsstoff aus Populus tremula Rinde und Blätter im Verhältnis 1 : 2 vorhanden sind.

**Claims**

1. Medicine with analgesic and anti-inflammatory effect made from plants with extracts or substances from
populus tremula
solidago virgaurea
fraxinus excelsior
as sole active substances.

2. Medicine according to claim 1, characterized in that it includes extracts or substances consisting of
50 - 70 Vol.-% of populus tremula
10 - 30 Vol.-% of solidago virgaurea
10 - 30 Vol.-% of fraxinus excelsior.

3. Medicine according to claim 1 or 2, characterized in that it includes extracts or substances consisting of
60 Vol.-% of populus tremula
20 Vol.-% of solidago virgaurea
20 Vol.-% of fraxinus excelsior.

4. Medicine according to claim 1 or 3, characterized in that bark and leaves are present in the ratio of 1 : 2 in the extract or substance of populus tremula.

## Revendications

1. Médicament à effet antalgique et antiinflammatoire à base de plantes avec des extraits ou des substances de
Populus tremula
Solidago virgaurea
Fraxinus excelsior
comme seules substances actives.

2. Médicament selon la revendication 1, caracterisé en ce qu'il contient des extraits ou des substances constitués de
50 - 70 % en volume de Populus tremula
10 - 30 % en volume de Solidago virgaurea
10 - 30 % en volume de Fraxinus excelsior.

3. Médicament selon la revendication 1 ou 2, caractérisé en ce qu'il contient des extraits ou des substances constitués de
60 % en volume de Populus tremula
20 % en volume de Solidago virgaurea
20 % en volume de Fraxinus excelsior.

4. Médicament selon l'une des revendications 1 à 3, caractérisé en ce que l'extrait ou substance de Populus tremula est obtenu a partir d'écorces et de feuilles dans le rapport 1 : 2.

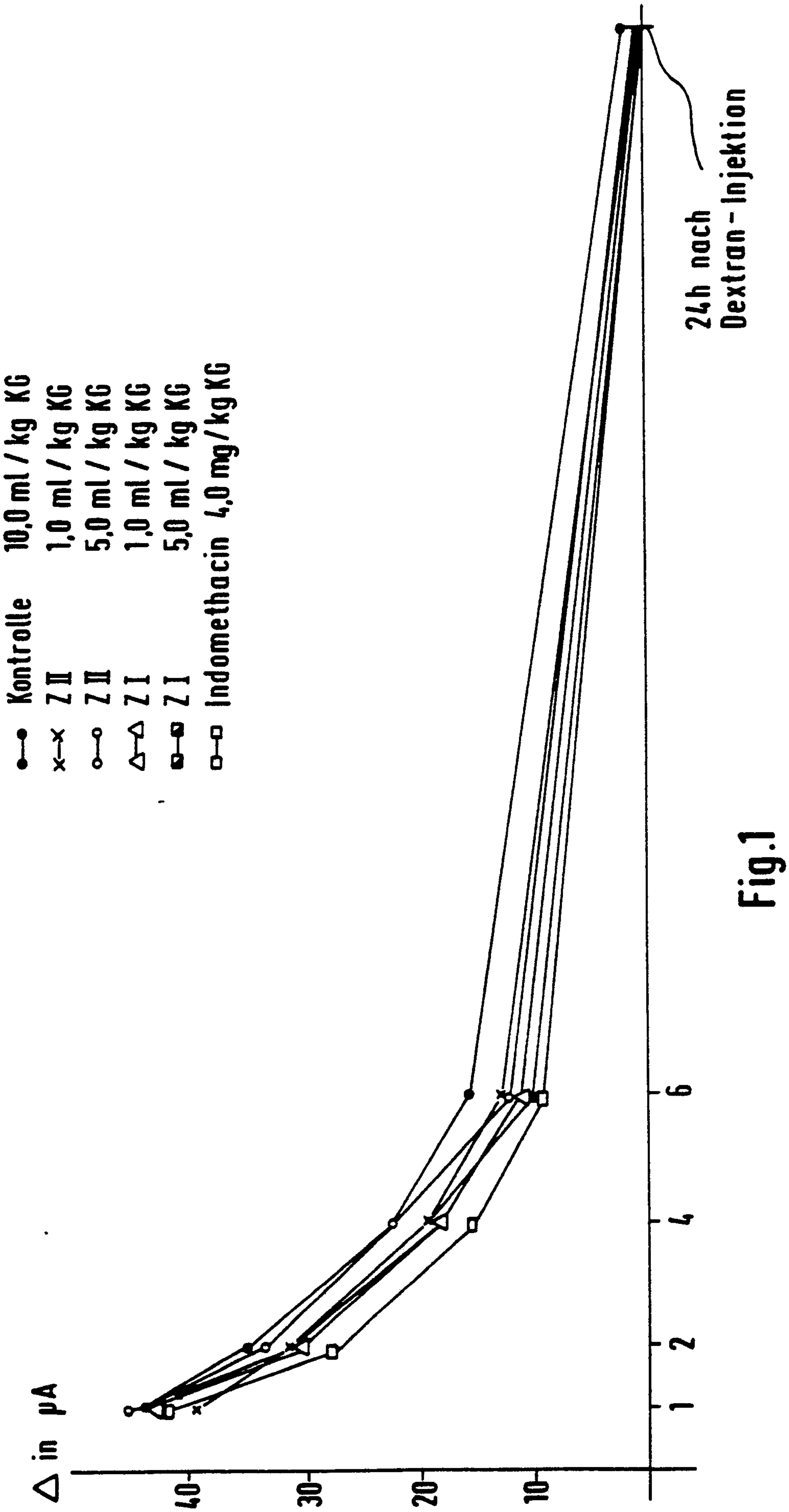
Δ in μA
40
30
20
10
1
2
4
6
Kontrolle 10,0 ml / kg KG
Z II 1,0 ml / kg KG
Z II 5,0 ml / kg KG
Z I 1,0 ml / kg KG
Z I 5,0 ml / kg KG
Indomethacin 4,0 mg/kg KG
24h nach
Dextran-Injektion

Fig.1

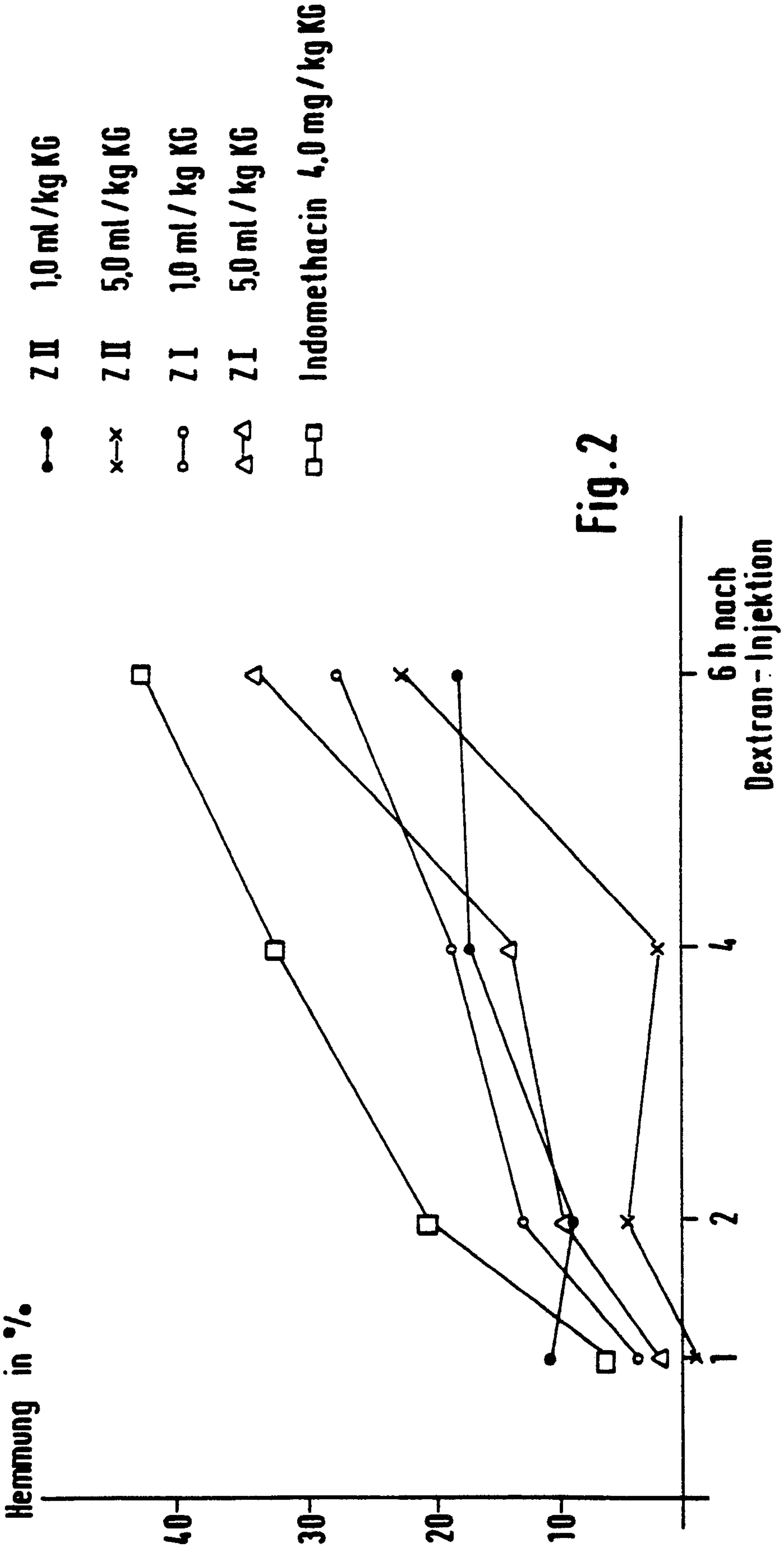
Hemmung in %
40
30
20
10
1
2
4
6 h nach
Dextran - Injektion
Z II 1,0 ml/kg KG
Z II 5,0 ml/kg KG
Z I 1,0 ml/kg KG
Z I 5,0 ml/kg KG
Indomethacin 4,0 mg/kg KG

Fig. 2

Fig.3

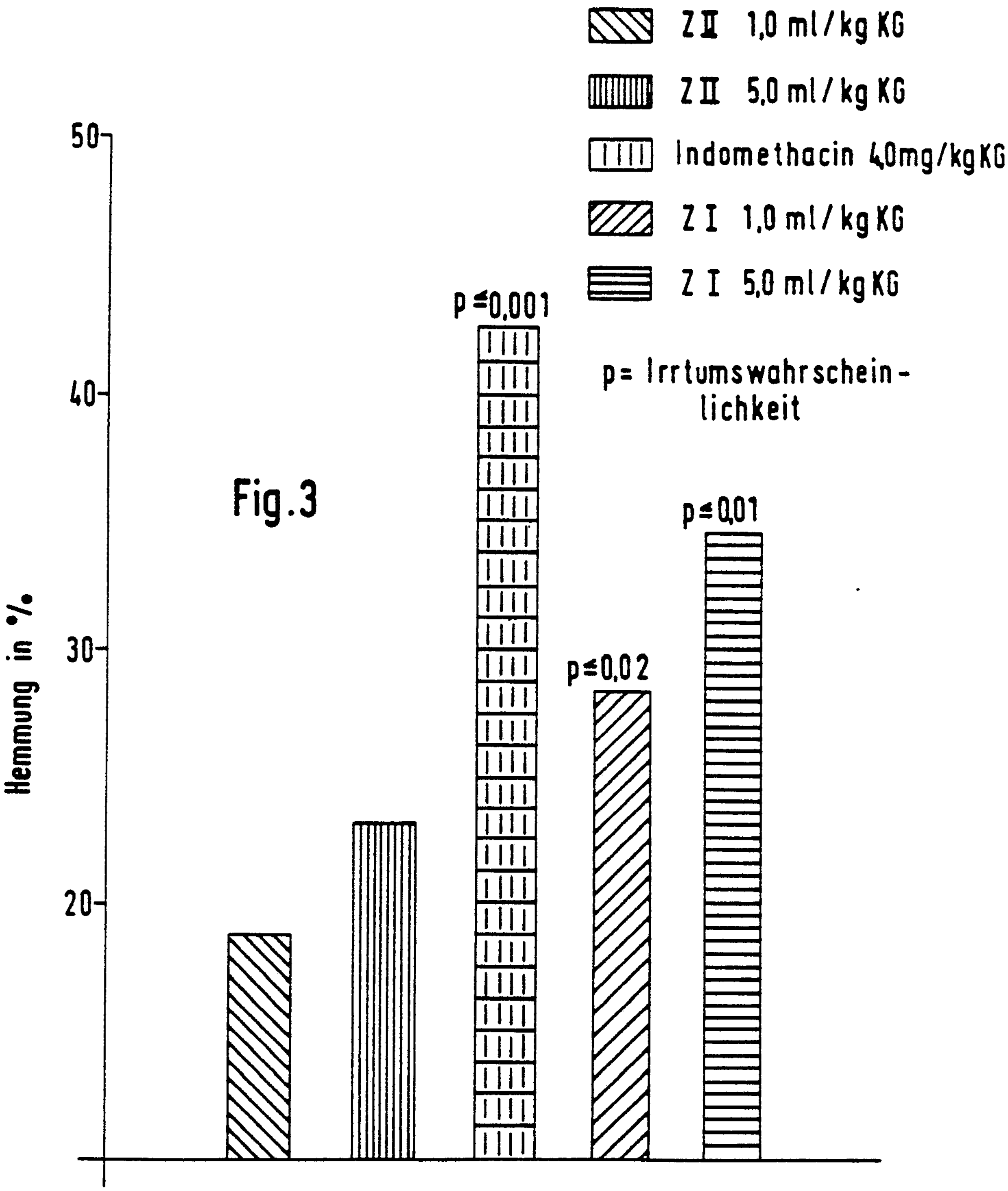
ZII 1,0 ml/kg KG
ZII 5,0 ml/kg KG
Indomethacin 4,0mg/kgKG
ZI 1,0 ml/kg KG
ZI 5,0 ml/kg KG
p= Irrtumswahrscheinlichkeit
p≤0,001
p≤0,02
p≤0,01
Hemmung in %
50
40
30
20